# FASCICULE DE BREVET EUROPEEN

(11) **EP 1 074 230 B1**
(45) Date de publication et mention de la délivrance du brevet: **22.09.2004**
(21) Numéro de dépôt: 00420173.7
(22) Date de dépôt: 03.08.2000
(51) Int. Cl.: A61F 2/42, A61B 17/17, A61F 2/46

(54) **Implant malléolaire pour prothèse partielle ou totale de cheville et matériel ancillaire de pose d'un tel implant**
Malleoläres Implantat für eine Teil- oder Totalknöchelprothese und Hilfsinstrument zum Einsetzen eines solchen Implantats
Malleolar implant for a partial or total ankle prosthesis and ancillary equipment for fitting such an implant

(30) Priorité: 05.08.1999 FR 9910340
(43) Date de publication de la demande: 07.02.2001
(60) Demande divisionnaire: 03027583.8
(73) Titulaire: Tornier, 38330 Saint-Ismier (FR)
(72) Inventeur: Tornier, Alain, 38330 Saint Ismier (FR); Bonnin, Michel, 69340 Francheville (FR); Colombier, Jean-Alain, 31130 Balma (FR); Judet, Thierry, 92410 Ville d'Avray (FR)
(74) Mandataire: Myon, Gérard Jean-Pierre

(56) Documents cités:
- EP-A- 0 864 304
- FR-A- 2 700 462
- US-A- 3 987 500
- US-A- 4 159 716
- US-A- 4 235 428
- US-A- 5 601 550

## Description

L'invention a trait à un implant malléolaire pour une prothèse partielle ou totale de la cheville et à un matériel ancillaire de pose d'un tel implant.

Il est connu, par exemple, de EP-A-0 864 304 d'équiper une prothèse de cheville d'un implant malléolaire destiné à venir en appui contre une surface articulaire au niveau de l'astragale, qu'il s'agisse d'une surface naturelle ou d'une surface d'un composant prothétique. Lors d'une intervention sur une cheville, l'accès aux surfaces articulaires internes est limité par le système ligamentaire qui ne permet pas forcément une luxation suffisante de l'articulation. En particulier, l'accès à la surface interne de la malléole péronière peut être insuffisant, ce qui induit des difficultés de positionnement et de mise en place de l'implant, notamment par impaction.

En référence aux modes de réalisations des figures 4 et 5, il est envisagé dans EP-A-0 864 304 d'introduire un implant à partir de la face externe du péroné. Cependant, ceci limite nécessairement la surface de la tête de cet implant, qui doit être inférieure ou égale à la surface de l'orifice prévu dans l'os, de telle sorte qu'elle est forcément de dimensions relativement faibles, sauf à fragiliser sensiblement la malléole.

Pour les raisons qui précèdent, la mise en place des implants malléolaires dans les prothèses connues ne donne pas entièrement satisfaction.

C'est à ces inconvénients qu'entend plus particulièrement remédier l'invention, en proposant un nouvel implant malléolaire qui peut être mis en place de façon précise, alors même que l'accès à la surface interne de la malléole péronière peut être limité et que sa tête articulaire a des dimensions lui permettant de remplir efficacement sa fonction.

Dans cet esprit, l'invention concerne un implant malléolaire comprenant une tête, destinée à venir en appui contre l'astragale ou un composant prothétique astragalien et une queue prévue pour être introduite dans un perçage du péroné, caractérisé en ce que cette queue est pourvue de moyens d'accrochage d'un organe de traction manoeuvrable à partir du côté extérieur du péroné, pour la mise en place de cette queue dans ce perçage.

Grâce à l'invention, l'implant peut être pré-positionné du côté de la face interne de la malléole péronière et tiré à travers le perçage prévu débouchant dans la malléole, de telle sorte que le chirurgien n'a pas à manipuler précisément l'implant à l'intérieur de l'articulation, c'est-à-dire entre le péroné et le tibia ou entre le péroné et l'astragale. Le chirurgien peut exercer sur l'organe de traction, qui peut être un lien flexible tel qu'un fil de suture, un effort efficace sans être gêné par des os environnants. Il en résulte que la mise en place de la queue de l'implant dans le perçage dans le péroné peut être précise, en particulier du fait que le diamètre extérieur de la queue peut être sensiblement égal au diamètre interne de ce perçage, car l'effort de traction qui peut être exercé par l'extérieur du péroné peut être intense.

Selon un aspect avantageux de l'invention, la queue est pourvue d'au moins un orifice de passage d'un lien flexible apte à être engagé à travers le perçage. En particulier, la queue peut comprendre plusieurs orifices de passage d'un lien flexible, ces orifices étant répartis sur la longueur de cette queue.

Selon un autre aspect avantageux de l'invention, la queue est pourvue de moyens de retenue axiale à l'intérieur du perçage. Ces moyens, qui peuvent être formés par des collerettes radiales externes réparties sur la longueur de la queue, permettent d'immobiliser efficacement la queue à l'intérieur du perçage après qu'elle y a été introduite par traction sur l'organe ou lien flexible.

L'invention concerne également un matériel ancillaire de pose d'un implant malléolaire tel que précédemment décrit et, plus spécifiquement, un matériel qui comprend un bloc-entretoise, apte à être inséré entre le tibia et l'astragale d'une cheville, et une patte solidaire de ce bloc-entretoise et s'étendant jusqu'au voisinage de la surface externe de la malléole péronière lorsque le bloc-entretoise est en place entre le tibia et l'astragale, cette patte supportant un guide de perçage de la malléole à partir de sa surface externe.

Grâce à l'invention, le perçage de la malléole péronière peut être effectué à partir de sa surface externe et en direction de sa surface interne, avec un positionnement relatif déterminé par rapport au tibia et à l'astragale, de telle sorte que la position de l'implant malléolaire en place dans ce perçage est déterminée avec précision par rapport aux surfaces articulaires respectives de l'astragale ou du tibia ou de composants prothétiques correspondants. Le bloc-entretoise peut être prévu pour coopérer avec des surfaces articulaires naturelles du tibia et/ou de l'astragale ou avec des surfaces créées par résection de ces os, dans le cas de la pose d'une prothèse totale de cheville.

Selon un aspect avantageux de l'invention, le bloc-entretoise est pourvu d'un logement de réception d'une cale d'épaisseur adaptée à l'écart entre les surfaces inférieure du tibia et supérieure de l'astragale. Cet aspect de l'invention permet de maintenir un écart, correspondant à celui qui sera ultérieurement crée par des éléments prothétiques montés en partie basse du tibia et en partie supérieure de l'astragale, lors de la détermination de la position de l'implant malléolaire.

Selon un autre aspect avantageux de l'invention, la patte est articulée sur le bloc-entretoise, avec une possibilité de pivotement limitée. Ceci permet d'ajuster la position du guide de perçage de la malléole autour de l'axe de pivotement de la patte par rapport au bloc-entretoise. Dans ce cas, le bloc-entretoise et la patte sont avantageusement pourvus d'orifices de passage d'un axe de pivotement commun.

On peut en outre prévoir que le guide de perçage est associé à un dispositif de serrage de la malléole contre une surface d'appui formée sur la patte ou le bloc-entretoise. Ceci permet une immobilisation ferme de la malléole péronière lors de son perçage et assure ainsi la précision de l'opération de perçage.

L'invention sera mieux comprise et d'autres avantages de celle-ci apparaîtront plus clairement à la lumière de la description qui va suivre d'un mode de réalisation d'un implant malléolaire et de son matériel ancillaire de pose conformes à l'invention, donnée uniquement à titre d'exemple et faite en référence aux dessins annexés dans lesquels :
- la figure 1 est une vue en perspective d'un implant conforme à l'invention ;
- la figure 2 est une vue en perspective de l'implant de la figure 1 en cours de mise en place dans une malléole péronière représentée avec arrachement ;
- la figure 3 est une représentation schématique de principe, avec arrachements partiels, d'un matériel ancillaire de pose de l'implant de la figure 1 en cours d'utilisation et
- la figure 4 est une vue en perspective par en dessous du matériel de la figure 1.

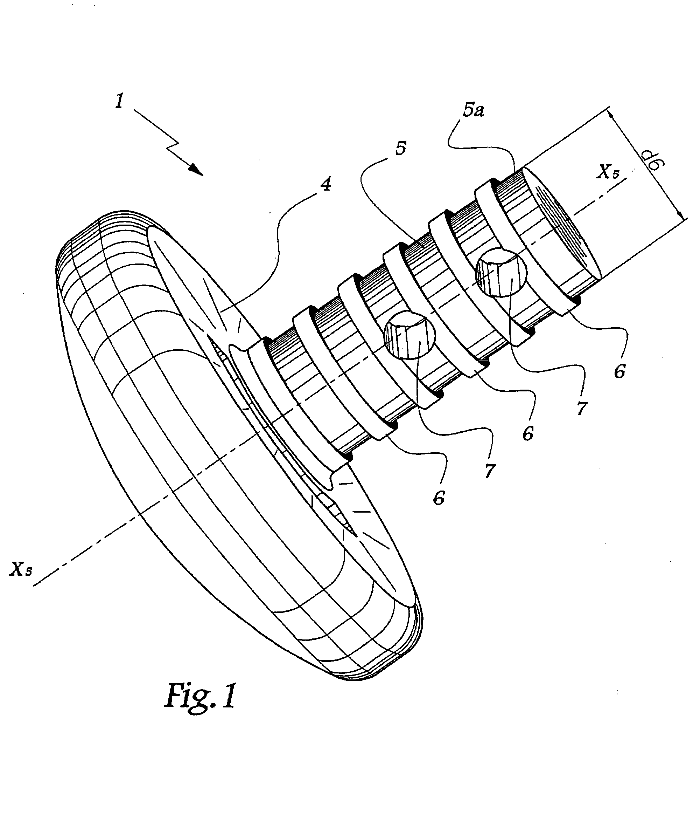

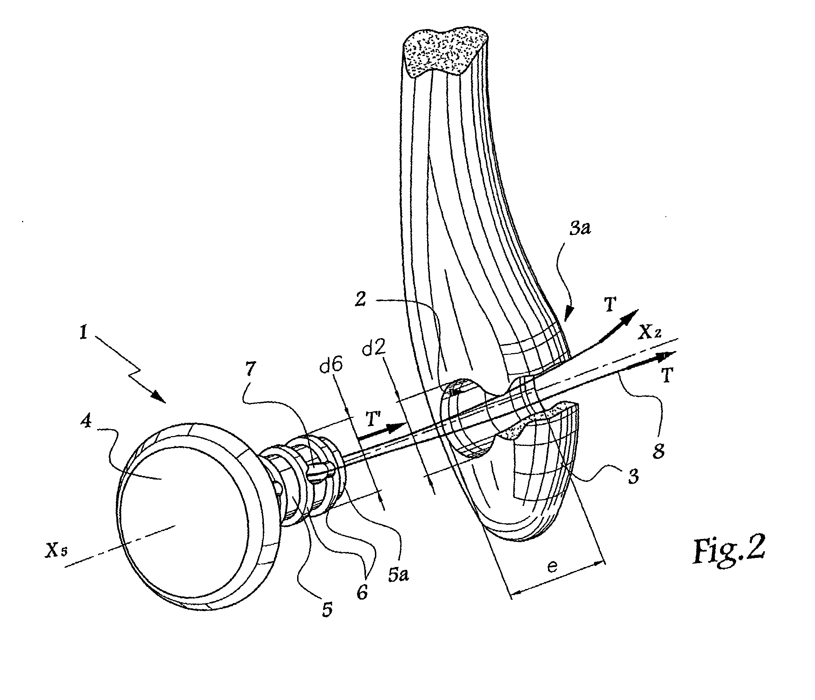

L'implant 1 représenté aux figures 1 et 2 est destiné à être introduit dans un perçage 2 ménagé dans la malléole externe ou péronière 3. L'implant 1 comprend une tête bombée 4, sensiblement selon une forme de calotte sphérique et dont le rayon de courbure est globalement égal à celui de la joue externe de l'astragale de la cheville considérée. La queue 5 de l'implant 1 est pourvue de collerettes radiales externes 6 dont le diamètre extérieur d₆ est sensiblement égale au diamètre interne d₂ du perçage 2.

Conformément à l'invention, deux orifices 7 sont prévus dans la queue 5 et sont susceptibles de recevoir un fil de suture 8 ou un autre lien flexible. Lorsqu'un tel fil est engagé dans l'un des orifices 7, il est possible d'exercer sur le fil 8, un effort de traction T qui est transmis par le fil 8 à la queue 5 comme représenté par la flèche T' à la figure 2. Ainsi, en tirant sur le fil 8, le chirurgien introduit la queue 5 dans le perçage 2 sans devoir exercer un effort de poussée sur la tête 4 qui peut être difficilement accessible du fait du système ligamentaire environnant.

En d'autres termes, il suffit au chirurgien de placer un fil dans un des orifices 7, de passer les deux brins du fil 8 dans le perçage 2 par la face interne de la malléole, puis de tirer les brins par le côté externe de la malléole. La traction sur le fil 8 a pour effet d'introduire la queue 5 de l'implant 1 dans le perçage 2 et de plaquer la tête 4 sur l'os. L'effort de traction T exercé sur le fil 8 peut être intense et dirigé parallèlement à l'axe longitudinale X₂ du perçage 2, de telle sorte que la queue est efficacement tirée vers l'intérieur du perçage 2. En particulier, compte tenu de la direction et de l'intensité de l'effort de traction T, on peut prévoir que les diamètres d₂ et d₆ sont sensiblement égaux, de telle sorte que la queue 5 est fermement maintenue en position après sa mise en place.

La queue 5 est pourvue de deux perçages 7 répartis selon son axe X₅, le perçage 7 le plus proche de l'extrémité 5a de la queue 5 étant utilisé. Le fait que la queue 5 comporte plusieurs orifices 7 permet d'utiliser un orifice 7 relativement proche de l'extrémité 5a de la queue 5 et de disposer d'un tel orifice y compris lorsque la queue 5 est sectionnée pour adapter sa longueur à l'épaisseur e de la malléole 3. Bien entendu, le nombre de perçages 7 peut être augmenté si nécessaire.

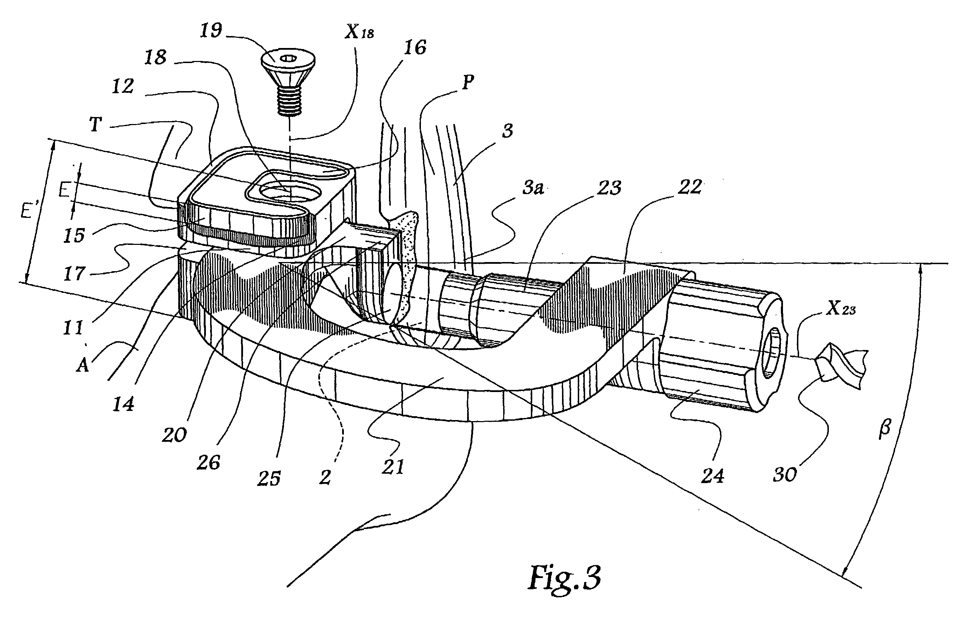

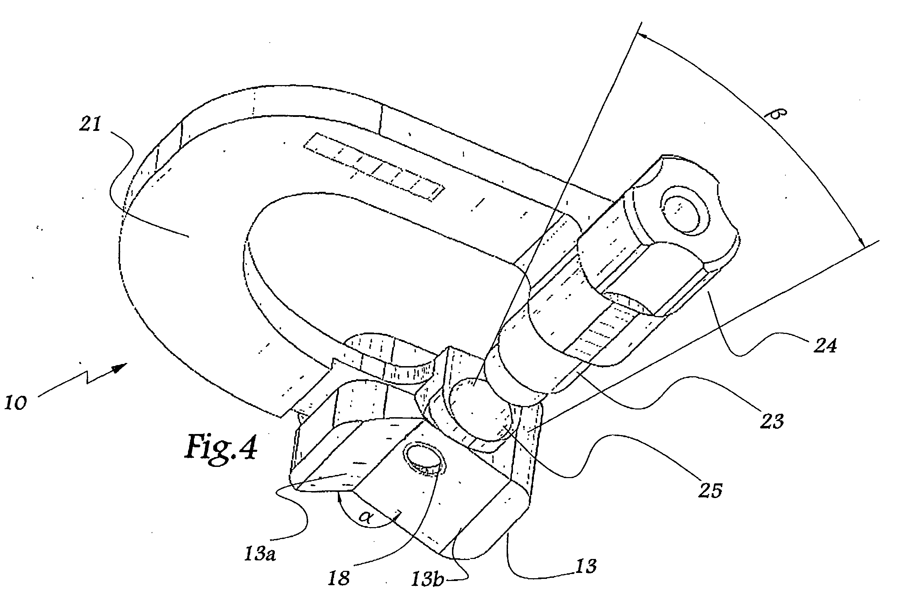

Le perçage 2 est réalisé par la face externe 3a de la malléole 3 grâce au matériel ancillaire représenté aux figures 3 et 4. Ce matériel 10 comprend un bloc-entretoise 11 prévu pour être disposé entre le tibia T et l'astragale A d'une cheville à équiper en implant 1. Le bloc 11 comprend une surface supérieure 12 sensiblement plane destinée à coopérer avec une surface plane créée par résection de la l'extrémité distale du tibia. La surface inférieure 13 du bloc 11 est formée de deux surfaces planes 13a et 13b inclinées l'une par rapport à l'autre d'un angle α, les surfaces 13a et 13b étant prévues pour venir respectivement en appui sur des surfaces correspondantes créées par résection de la patte supérieure de l'astragale A.

La surface 12 du bloc 11 comprend un logement 14 en forme de C destiné à recevoir une cale 15 dont la surface supérieure 16 est en contact avec la surface inférieure du tibia T. L'épaisseur E de la cale 15 représentée à la figure 3 est telle que sa surface supérieure 16 est affleurante avec la surface supérieure 12 du bloc 11.

Cependant, des cales d'épaisseur plus importante peuvent être utilisées lorsque l'écart E' entre les surfaces inférieure du tibia et supérieure de l'astragale est plus important que dans la configuration représentée à la figure 3.

Le bloc 11 définit un logement 17 de réception de l'extrémité 20 d'une patte 21 globalement en forme de C. L'extrémité 20 est pourvue d'un perçage non représenté qui, dans la configuration des figures 3 et 4, est aligné avec un perçage 18 ménagé dans le bloc 11 et traversant ce bloc de haut en bas, c'est-à-dire reliant les surfaces 12 et 13. Une vis 19 peut être introduite dans ce perçage qui est au moins partiellement taraudé, ce qui permet d'immobiliser l'extrémité 20 de la patte 21 à l'intérieur du logement 17. En pratique, le jeu ménagé lors du serrage de la vis 19 autorise un pivotement limité autour de l'axe X₁₈ du perçage 18.

Au niveau de son extrémité 22 opposée à l'extrémité 20, la patte 21 supporte un système de serrage 23 manoeuvrable grâce à une mollette 24 et permettant de plaquer la malléole 3 du péroné P contre une butée 25 formée sur une extension 26 de l'extrémité 20 de la patte 21. On note X₂₃ l'axe longitudinal de ces moyens de serrage. Les moyens de serrage 23 sont creux, de telle sorte qu'un forêt 30 peut être introduit jusqu'au niveau de la face externe 3a de la malléole 3 pour réaliser le perçage 2 depuis l'extérieur vers l'intérieur de la malléole 3. Ainsi, le chirurgien peut aisément viser la partie adéquate de la malléole 3 grâce aux moyens de serrage 23 qui constituent également un guide de perçage pour le forêt 30.

Comme la patte 21 est susceptible de pivoter autour de l'axe X₁₈, la position de l'axe X₂₃ est variable en pivotement autour de cet axe X₁₈, ce qui permet d'ajuster au mieux l'orientation du perçage 2 en fonction de la géométrie exacte de la malléole 3. On note β l'angle de pivotement maximum de l'axe X₂₃ autour de l'axe X₁₈. En pratique, l'angle β est de l'ordre de 10°.

Grâce au matériel 10, on peut donc former, par l'extérieur, un perçage 2 permettant une implantation rapide et efficace de l'implant 1.

Lorsque des cales 15 de hauteur supérieure à celles représentées à la figure 2 sont utilisé, il est possible de prévoir qu'elles viennent en recouvrement du perçage 18 car la vis 19 est mise en place avant le positionnement de la cale 15 qui est effectué en cours d'opération en fonction de l'écart E'.

L'invention a été représentée avec une prothèse totale de cheville, ce qui correspond à la géométrie des surfaces 12 et 13 du bloc 11. Elle est cependant applicable à une prothèse partielle de cheville, sans modification de l'implant 1, le matériel ancillaire étant alors adapté à la géométrie des surfaces anatomiques d'articulation entre le tibia et l'astragale.

## Revendications

1. Implant malléolaire (1) pour prothèse partielle ou totale de cheville comprenant une tête (4), destinée à venir en appui contre l'astragale (A) ou un composant prothétique astragalien, et une queue (5), prévue pour être introduite dans un perçage (2) du péroné (P), **caractérisé en ce que** ladite queue est pourvue de moyens (7) d'accrochage d'un organe de traction (8) manoeuvrable à partir du côté extérieur (3a) du péroné, pour la mise en place de ladite queue dans ledit perçage.

2. Implant selon la revendication 1, **caractérisé en ce que** ladite queue (5) est pourvue d'au moins un orifice (7) de passage d'un lien flexible (8) apte à être engagé à travers ledit perçage (2).

3. Implant selon la revendication 2, **caractérisé en ce que** ladite queue (5) est pourvue de plusieurs orifices (7) de passage d'un lien flexible (8), lesdits orifices étant répartis sur la longueur de ladite queue.

4. Implant selon l'une des revendications précédentes, **caractérisé en ce que** ladite queue (5) est pourvue de moyens de retenue axiale (6) à l'intérieur dudit perçage (2).

5. Implant selon la revendication 4, **caractérisé en ce que** lesdits moyens de retenue sont formés par des collerettes radiales externes (6) réparties sur la longueur de ladite queue (5).

6. Matériel ancillaire (10) de pose d'un implant malléolaire (1) selon l'une des revendications précédentes, **caractérisé en ce qu'**il comprend un bloc-entretoise (11), apte à être inséré entre le tibia (T) et l'astragale (A) d'une cheville, et une patte (21) solidaire dudit bloc-entretoise et s'étendant jusqu'au voisinage de la surface externe (3a) de la malléole péronière (3) lorsque le bloc-entretoise est en place entre le tibia et l'astragale, ladite patte supportant un guide de perçage (23) de la malléole à partir de sa surface externe.

7. Matériel selon la revendication 6, **caractérisé en ce que** ledit bloc-entretoise (11) est pourvu d'un logement (14) de réception d'une cale (15) d'épaisseur (E) adaptée à l'écart (E') entre les surfaces inférieure du tibia (T) et supérieure de l'astragale (A).

8. Matériel selon l'une des revendications 6 ou 7, **caractérisé en ce que** ladite patte (21) est articulée (en 18) sur ledit bloc-entretoise (11) avec une possibilité de pivotement (β) limitée.

9. Matériel selon la revendication 8, **caractérisé en ce que** ledit bloc-entretoise (11) et ladite patte (21) sont pourvus d'orifices (18) de passage d'un axe de pivotement (19) commun.

10. Matériel selon l'une des revendications 6 à 9, **caractérisé en ce que** ledit guide de perçage est associé à un dispositif de serrage (23) de la malléole (3) contre une surface d'appui (25) formée sur ladite patte (21) ou ledit bloc-entretoise (11).

## Patentansprüche

1. Knöchelimplantat (1) für teilweise oder komplette Knöchelprothese mit einem Kopf (4), der dazu bestimmt ist, gegen den Talus (A) oder einen Talusprothesenbestandteil zum Aufliegen zu kommen, und einem Stiel (5), der dazu vorgesehen ist, in eine Bohrung (2) des Wadenbeins (P) eingeführt zu werden, **dadurch gekennzeichnet, dass** der Stiel mit Mitteln (7) zum Anhängen eines Zugorgans (8) versehen ist, das von der Außenseite (3a) des Wadenbeins zum Anbringen des Stiels in der Bohrung manövrierbar ist.

2. Implantat nach Anspruch 1, **dadurch gekennzeichnet, dass** der Stiel (5) mit mindestens einer Öffnung (7) zum Durchgehen eines biegsamen Bands (8) vorgesehen ist, das durch die Bohrung (2) eingeführt werden kann.

3. Implantat nach Anspruch 2, **dadurch gekennzeichnet, dass** der Stiel (5) mit mehreren Öffnungen (7) zum Durchgehen eines biegsamen Bands (8) versehen ist, wobei die Öffnungen auf die Länge des Stiels verteilt sind.

4. Implantat nach einem der vorhergehenden Ansprüche, **dadurch gekennzeichnet, dass** der Stiel (5) mit axialen Rückhaltemitteln (6) im Inneren der Bohrung (2) versehen ist.

5. Implantat nach Anspruch 4, **dadurch gekennzeichnet, dass** die Rückhaltemittel aus radialen äußeren Kragen (6) gebildet sind, die über die Länge des Stiels (5) verteilt sind.

6. Hilfsmaterial (10) zum Anbringen eines Knöchelimplantats (1) nach einem der vorhergehenden Ansprüche, **dadurch gekennzeichnet, dass** es einen Block-Abstandshalter (11) umfasst, der zwischen das Schienbein (T) und den Talus (A) eines Knöchels eingefügt werden kann, und eine Klammer (21), die fest mit dem Block-Abstandshalter verbunden ist und sich bis in die Nähe der Außenfläche (3a) des Wadenbeinknöchels (3) erstreckt, wenn der Block-Abstandshalter zwischen dem Schienbein und dem Talus angebracht ist, wobei die Klammer eine Bohrführung (23) des Knöchels ausgehend von seiner Außenfläche stützt.

7. Material nach Anspruch 6, **dadurch gekennzeichnet, dass** der Block-Abstandshalter (11) mit einer Aufnahme (14) zum Aufnehmen eines Keils (15) mit Stärke (E) vorgesehen ist, der an den Abstand (E') zwischen der unteren Fläche des Schienbeins (T) und der oberen Fläche des Talus (A) angepasst ist.

8. Material nach einem der Ansprüche 6 oder 7, **dadurch gekennzeichnet, dass** die Klammer (21) (in 18) auf dem Block-Abstandshalter (11) mit einer eingeschränkten Schwenkmöglichkeit (β) angelenkt ist.

9. Material nach Anspruch 8, **dadurch gekennzeichnet, dass** der Block-Abstandshalter (11) und die Klammer (21) mit Öffnungen (18) zum Durchgehen einer gemeinsamen Schwenkachse (19) versehen sind.

10. Material nach einem der Ansprüche 6 bis 9, **dadurch gekennzeichnet, dass** die Bohrführung mit einer Spannvorrichtung (23) des Knöchels (3) gegen eine Auflagefläche (25) versehen ist, die auf der Klammer (21) oder dem Block-Abstandshalter (11) gebildet ist.

## Claims

1. Malleolar implant (1) for partial or total ankle prosthesis comprising a head (4) intended to bear on the talus (A) or a prosthetic talus component, and a tail (5), intended to be introduced into a drilled hole (2) in the fibula (P), **characterised in that** that the said tail is provided with means (7) for hooking in a traction member (8) that can be manoeuvred from the outside (3a) of the fibula in order to fit the said tail in the said drilled hole.

2. Implant according to Claim 1, **characterised in that** the said tail (5) is provided with at least one opening (7) for passing through it a flexible tie (8) suitable for engaging through the said drilled hole (2).

3. Implant according to Claim 2, **characterised in that** the said tail (5) is provided with several openings (7) for passing through them a flexible tie (8), the said openings being distributed along the length of the said tail.

4. Implant according to one of the preceding claims, **characterised in that** the said tail (5) is provided with means (6) for axial retention inside the said drilled hole (2).

5. Implant according to Claim 4, **characterised in that** the said retention means are made up of external radial collars (6) distributed along the length of the said tail (5).

6. Ancillary equipment (10) for fitting a malleolar implant (1) according to one of the preceding claims, **characterised in that** it comprises a bracing block (11), suitable for insertion between the tibia (T) and the talus (A) of an ankle, and a lug (21) integral with the said bracing block and extending to the vicinity of the external surface (3a) of the fibular malleolus (3) when the bracing block is in place between the tibia and the talus, the said lug supporting a guide (23) for drilling the malleolus from its external surface.

7. Equipment according to Claim 6, **characterised in that** the said bracing block (11) is provided with a seat (14) for accommodating a block (15) of thickness (E) matched to the spacing (E') between the lower surface of the tibia (T) and the upper surface of the talus (A).

8. Equipment according to one of Claims 6 or 7, **characterised in that** that said lug (21) is articulated (at 18) on the said bracing block (11) with a limited possibility of pivoting (β).

9. Equipment according to Claim 8, **characterised in that** the said bracing block (11) and the said lug (21) are provided with openings (18) for passing a common pivot pin (19) through them.

10. Equipment according to one of Claims 6 to 9, **characterised in that** the said drilling guide is associated with a device (23) for locking the malleolus (3) against a bearing surface (25) formed on the said lug (21) or the said bracing block (11).
